# EUROPEAN PATENT APPLICATION

(11) **EP 0 610 756 A1**
(43) Date of publication of application: **17.08.1994**
(21) Application number: 94101293.2
(22) Date of filing: 28.01.1994
(51) Int. Cl.: B01D 67/00, B01D 71/56

(54) **Activated membrane and method for ligate separation**

(30) Priority: 11.02.1993 GB 9302755
(71) Applicant: Pall Corporation, East Hills, New York 11548 (US)
(72) Inventor: Ball, Peter Robert, Dr., Langrish, Hampshire, GU32 1PH (GB); Cooper, Graham Alan Ernest, Purbrook, Hampshire, P07 5JU (GB)
(74) Representative: Geissler, Bernhard, Dr.

(57) **Abstract**

An activated membrane and method for preparing same are provided which comprises reacting an acceptor molecule prior to its attachment to the membrane with a protector molecule to form a complex. The complex is then attached to the membrane and the protector molecule subsequently removed. The membrane activated in this manner is used in a method for recovering a ligate from solution using affinity separation techniques. The immobilization or binding capacity is significantly increased with the membranes and methods disclosed herein.

## Description

The present invention relates to a method for preparing activated membranes suitable for immobilizing ligate. The invention also relates to the activated membrane obtained by the method which is useful in affinity separation procedures. The invention further relates to a method for binding a mobile target ligate to a membrane surface having an immobilized ligand attached to it which is capable of reversible complex formation with the mobile target ligate.

The term "affinity separation" is used to describe a process by which immunoglobulins, proteinaceous substances or other biologically active substances capable of biospecific complex formation are extracted from a fluid. Such substances are referred to herein as "ligates". A common procedure involves passing the fluid through a packed column containing a porous material, for example in the form of porous beads or a porous membrane, which has a covalently bound acceptor molecule capable of reversibly immobilizing the ligate through complex formation. The acceptor molecule will be the biospecific pair molecule to the above mentioned ligates. These molecules bound to the membrane surface are sometimes referred to herein as "ligands " or "ligand molecules" to distinguish them from ligates to be separated from solution.

It is well-known, for example, that Protein A as the acceptor or ligand molecule binds to the Fc region of immunoglobulins of the class IgG at neutral pH values. Thus a porous membrane with Protein A bound to its surface can be used to separate IgG specifically from a fluid when it is passed through the membrane. The Protein A/IgG complex can be subsequently broken, providing a method for purification of IgG.

Despite advances in this technique a need exists for improved membrane materials and improved methods of separation. In view of the surface area of the porous materials involved, and the amount of acceptor molecule thought to be attached to the surfaces, a higher capacity of ligate separation might be expected than is presently found. One reason for the low separation capacity might be that the acceptor ligand molecules are not always attached to the membrane surface in a manner which allows them to react with and immobilize the ligate. It would therefore be highly desirable to develop improved membranes and methods of separation by which the amount of ligate separated per gram of porous membrane employed is enhanced.

The object of the present invention is therefore to provide a method for preparing an improved membrane suitable for use in mobile ligate separation. A further object is to provide an activated membrane which is more efficient in binding ligates on its surface. A further object of the invention is to provide a method of ligate separation by which the binding capacity is increased. An even further object of the invention is to provide a system capable of sustaining high flow rates, for example filter membrane systems.

In accordance with the present invention, a method of preparing an activated membrane is provided as defined in the claims. The method comprises initially reacting an acceptor molecule, preferably a proteinaceous substance, with a protector molecule to form a complex. Thus before the acceptor molecules are attached to the membrane, their active sites are protected in the sense that the sites are occupied through complex formation with the protector molecule. During the subsequent attachment or loading of the membrane with the acceptor/protector complex, the activated sites will not effectively react or interact with the chemistry of the membrane surface.

The formed complex is then reacted with a porous, preferably microporous, membrane having active sites capable of binding with said complex. Such membranes are preferably ones which have been previously reacted with an activating agent to produce active sites on the membrane surface. These sites will be capable of binding with the acceptor molecule part of the complex. In a further, optional step of the method, the protector molecule is removed, leaving the acceptor molecule bound to the membrane. Preferably the protector molecule is removed by washing the membrane with an elution solution.

In one preferred embodiment, the protector ligand molecule is the ligate itself or a similar substance which is to be subsequently extracted in the affinity separation technique. For example, if the acceptor molecule is an antibody, the protector molecule could be the specific antigen or if the acceptor molecule is Protein A, the protector molecule could be IgG, and so on. In a more preferred embodiment, the protector molecule is a polysaccharide.

In accordance with the present invention, a porous membrane prepared by the above method is also provided as defined in the claims. The porous membrane used in preparing the activated membrane of the invention is preferably a microporous polyamide membrane which has been reacted with an activating agent to produce a linking molecule, which in turn is capable of binding with the acceptor/protector complex. More specifically, the activating agent or the product of its reaction with the membrane binds with the acceptor molecule and thus immobilizes the complex.

In accordance with a further embodiment of this invention a method or process for separating biologically separable, particularly biologically active, substances from solutions containing these, is provided. This method is defined in the claims.

Preferably a prefiltered impure solution is the starting material. These impure solutions may consist of culture supernatants in general and in particular tissue culture supernatants from which the cells etc. have been removed by prefiltration, preferably to 0.2 µm.

The impure solution is applied to the separation material or device in accordance with this invention. The separation material or device contains the activated sites defined herein. Upon contact with the impure solution, biologically separable substances will be attached to those sites, thereby removing these substances from the impure solution.

Usually the impure solution contains 0.01 to 0.1, typically about 0.05 mg/ml of biologically active substance. This solution is applied to the device or membrane defined herein. For example, a filter cartridge of 5 cm diameter, 5 cm high is employed at a flow rate of preferably 0.1 to 5, more particularly about 1 l/min.. Thereby, 10 to 500 mg, typically 50 to 100 mg of biologically active material is bound to this filter medium of the cartridge. This filter medium is then washed with a phosphate buffer solution to remove impurities. The bound biologically active material is then eluted using another buffer, for example glycine/acetic acid mixture in the case of IgGs, to give a much more concentrated solution of relatively pure IgG, for example a solution with an IgG concentration of 1 mg/ml. Preferably, this more concentrated solution is then further processed to remove the biologically separable, in particular the biologically active substances to obtain a solid material.

Preferably, the device is then washed, in particular with phosphate buffer solution to make it ready for a further purification from a prefiltered impure solution. It is envisaged that this cycle may be repeated tens of times, possibly with an intermediate laundering process where the device is washed, for example, with 4M urea solution at a pH of 6 to remove unwanted contaminating protein and the like which might interfere with the separation process described above.

Thus, the preferred method involves a plurality of cycles, each comprising
- a separation period, in which the impure solution is put into contact with the separation material or device or membrane as herein defined, preferably the solution is passed through the separation material or device or membrane,
- a wash period, in which the separation material device or membrane is washed substantially free of said solution while maintaining a substantial quantity of the biologically separable substance on the separating material or device or membrane,
- an elution period, in which the biologically separable substance is removed from the separating material or device or membrane, preferably to obtain a more concentrated solution of the separable material,
- optionally, an intermediate laundering step, in which the separating material or device or membrane is subjected to a stronger cleaning operation, in particular to remove unwanted or contaminating protein and the like from the separating material or device or membrane.

Preferably 10 to 100 of these cycles are performed with the same separating material or device or membrane before it is discarded.

The present invention also provides a method for recovering a ligate from a solution as defined in the claims. A ligate solution is passed through the activated porous membrane, where the acceptor molecule is capable of immobilizing or binding the ligate through the formation of a biospecific complex. The ligate solution may be a biological fluid of any type or a body fluid or constituent parts thereof, for example blood, serum, plasma or the like.

After binding to the acceptor molecule on the surface of the activated membrane, the so-immobilized ligate can optionally be eluted. Suitable elution procedures are known to those skilled in this art.

Further embodiments and advantages of the present invention will become apparent in the following description in conjunction with the drawings.

Figure 1 shows a reaction scheme representing the conventional procedure for attaching an acceptor molecule, in this case Protein A, to a membrane and separating a ligand, in this case IgG.

Figure 2 shows a reaction scheme according to a preferred embodiment on the present invention, where the acceptor molecule is Protein A and the protector molecule is IgG.

Figure 3 shows the binding capacity of variously activated membranes according to the invention as a function of the mole ratio of the protector molecule to acceptor molecule.

Figure 4 shows the IgG separation capacity as a function of the pore size of the membrane.

Figure 5 shows the separation or binding capacity of a membrane according to one embodiment of the invention compared to a known membrane as a function of the flow rate of ligand solution.

The preferred porous membrane employed in the method of preparing an activated membrane according to the invention is a polyamide membrane with controlled surface properties. Such membranes are surface-modified to contain chemically functional groups in high concentration at their surface. "Surface" in the present context is understood to mean the entire surface of the porous membrane including the exposed internal surfaces of the pores. The functional groups can include hydroxyl, carboxyl, amine or imine groups.

Particularly preferred for use in the present invention is a surface-modified, microporous polyamide membrane disclosed in the European patent application EP-A-272 841, EP-A-272 842 and US 4,968,533. The membrane is available from Pall Corporation under the name Bio-Inert®. The membrane is modified at its surface to become rich in hydroxyl groups to provide a low affinity for amide group-containing materials, particularly proteinaceous materials. This property is particularly advantageous for a clean separation when the ligand is a protein, as will be discussed below.

To provide a link or bond to the acceptor molecule, such surface-modified membranes are treated with an activating agent. The activating agent employed will depend upon how the porous membrane has been prepared, i.e. what functional groups are present. The reaction with an activating agent results in the formation of a linking molecule which, in turn, is capable of reacting with and immobilizing biologically active materials such as the acceptor ligand molecules. Such membranes are sometimes referred to as "biologically activated membranes". The particular procedure employed for forming the biologically activated membrane will depend on the choice of activating agents. Any suitable such membrane can be used in the present invention.

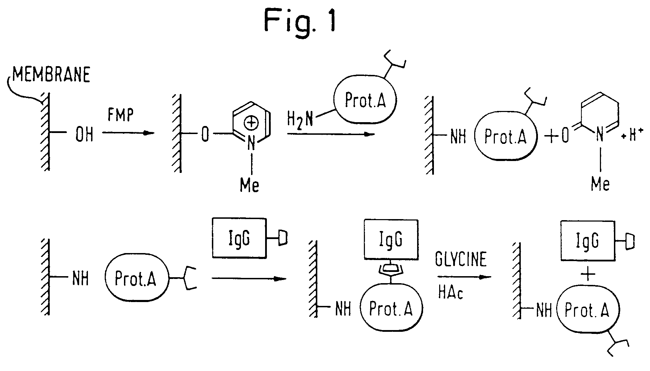

One method for activating the membrane is shown in Figure 1. The membrane is placed in a suitable container in contact with a solution of the activating agent and an appropriate solvent. In a preferred embodiment the activating agent is fluoromethylpyridinium tosylate (FMP) which is applied in acetonitrile as the solvent. As shown in Figure 1, FMP reacts with the OH-groups on the membrane surface creating an activated site where the acceptor molecule, in Figure 1 Protein A, can attach to the membrane. As will be explained below, however, the invention contemplates attaching an acceptor/protector molecule complex to the membrane. The biologically activated membrane prepared in this manner is the membrane for use as a starting material in the present invention. The remaining steps of Figure 1 represent procedures known in this art.

The porous membrane material, biologically activated as described above, is preferably a nylon membrane on a supporting substrate. The membrane material preferably has a BET surface area in the range of 200 to 600 cm²/cm², more preferably in the range of 300 to 500 cm²/cm². This high surface area is particularly desirable for the ligand recovery method of the present invention. The pore rating of the porous membrane is preferably within the range of 0.2 to 3.0 µm.

The method of preparing an activated membrane according to the present invention involves a procedure for more efficiently attaching the acceptor molecule, in Figure 1 Protein A, to the biologically activated porous membrane. "More efficiently" is to be broadly understood in that the acceptor molecule when attached according to the present method is capable of a higher capacity of ligate separation. The precise reason why previous methods and membranes have been less efficient is not understood. The acceptor molecule may have interacted with one or more of the activated sites on a membrane surface or other types of functional groups rendering its capacity for biospecific complex formation inactive.

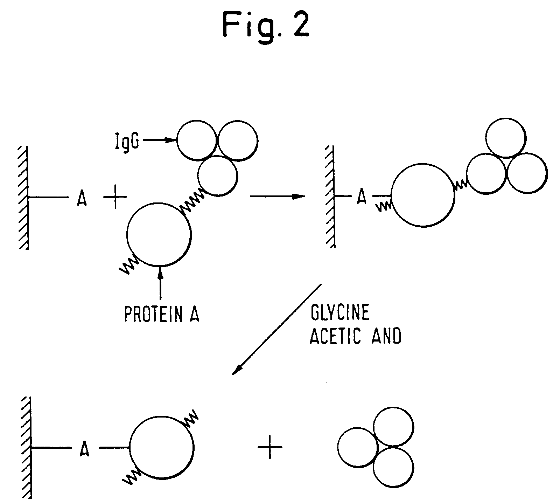

According to the present invention it has been found to be advantageous to protect at least one of the binding sites of the acceptor molecule prior to its attachment to the membrane. The protection is provided by complex formation of the acceptor molecule with a protector molecule. As can be seen in the embodiment in Figure 2, where the acceptor molecule is Protein A, one of the active regions of Protein A is used to form a complex with IgG. This complex is then contacted with the active site of the membrane. In this manner, the at least one active region of Protein A is not susceptible to interaction or any type of bonding with an active site of the membrane during the process of attachment to the membrane.

After Protein A, or the Protein A/IgG complex, is bound to the active site of the membrane, the protector molecule (IgG) is removed by known procedures, for example by elution. The resultant membrane then has the acceptor molecule attached to it in a form or in an environment which allows it to more efficiently immobilize a ligate. This is demonstrated by the fact that the activated membrane of this invention provides a higher separation capacity as will be shown in the examples below.

The acceptor molecule useful in the present invention can be any biological or organic molecule which reacts with a ligate by means of biospecific complex formation. Preferably the acceptor molecule is selected from the group of antibodies, antigenic substances, glycoproteins, Protein A, Protein G, lectins, carbohydrates, enzymes, co-factors, inhibitors, hormones, IgG class of immunoglobulins, carrier proteins, receptors, heparin, coagulation factors, histones and mimetic ligands. The acceptor molecule particularly preferred is Protein A. The acceptor molecule employed, the associated activating agent and the type of porous membrane employed will all depend on the particular application. The principle of the invention, however, remains that the acceptor molecule is first complexed with a protector molecule prior to attachment to the membrane.

The protector molecule can be selected from the group of substances to which the particular acceptor molecule is specific. For example, if the acceptor molecule is an antibody, the protector molecule can be the antigen to which it is specific. In many cases, the protector molecule will be the ligate itself which is later to be separated in an affinity separation procedure. In general, the protector molecule is selected from the group consisting of gelatins, polysaccharides, antibodies, antigenic substances, glycoproteins, Protein A, lectins, carbohydrates, enzymes, cofactors, inhibitors, hormones, IgG class of immunoglobulins, carrier proteins, receptors, heparin, coagulation factors and histones. Preferably the protector molecule is a polysaccharide, such as dextran, modified celluloses, etc. Most preferred is dextran.

When forming the acceptor/protector molecule complex it has been found particularly advantageous to form a mixture of the two substances in a mole ratio of protector molecule to acceptor molecule in the range of 1:0.5 to 1:1.5, more preferably a mole ratio in the range of 1:0.8 to 1:1.2. The ratios of the number of protector molecules to acceptor molecules can also depend on the number of reactive sites of the respective molecules capable of forming complex formation. The mixture, normally in a solvent, is allowed to react under suitable conditions to form a complex.

One preferred acceptor/protector pair is Protein A/IgG. The most preferred pair is Protein A with dextran being the protector molecule. In this embodiment it is advantageous when the molecular weight of dextran is selected to be within the range of 75 to 125 % of the molecular weight of the ligate to be subsequently separated. For example, if the acceptor molecule is Protein A and IgG is the ligate subsequently to be separated, the dextran employed should have a molecular weight in the range of 75 to 125 % of IgG. In a preferred embodiment, the molecular weight of dextran should be in the range of 90 to 110 % of the molecular weight of the ligand to be separated. This molecular weight effect has been found to increase the separation capacity of the membrane.

When reacting the complex with the porous membrane the complex can be contacted by immersion or any suitable means known in the art. The complex is normally contained in a solvent solution. In a particularly preferred embodiment, the acceptor/protector complex is contacted with the membrane in a volume of solution in an amount in the range of 80 to 120 % of the total void volume of the membrane. In other words, the amount of contacting solution containing the complex corresponds roughly to the void volume of the membrane pores. In a particularly preferred embodiment the amount of complex solution corresponds to 95 to 105 % of the total void volume of the membrane.

The following examples are intended to illustrate the advantages of the invention without limiting the invention to the embodiments disclosed therein.

All membranes used in the examples are the "Bio-Inert®" membrane available from Pall Corporation, disclosed in US patent 4,968,533 and the European application EP-A-272 841 and EP-272 842.

### Example I

This example is a comparative run which demonstrates the removal capacity or purification capacity of a membrane which has not been activated according to the present method. A Bio-Inert® membrane with a pore size of 0.2 µm was activated using fluoromethylpyridinium tosylate (FMP). Ten discs of the membrane material with a diameter of 47 mm (1g of membrane material) were impregnated with 6 mg/ml FMP in acetonitrile containing 1% triethylamine for 45 minutes. Activation with FMP as the activating agent was then followed by washing with acetonitrile, drying and then treating the membrane with Protein A (6 mg/ml) in phosphate buffered saline (PBS) for 1 hour. This step was followed by a treatment with 1% ethanolamine at a pH of 9.5 for 1 hour to remove FMP residues which have not reacted with Protein A and convert them to OH-groups. An optional step following this is to treat the membrane with 4 M urea at pH 6 in PBS to remove loosely bound Protein A. The discs were then finally washed again with PBS solution.

A solution of 10 mg of rabbit IgG was prepared in 20 ml of PBS. This solution was prefiltered with a Bio-Inert® filter having a pore size rating of 0.2 µm, the filter in this case serving as a true filtration element, i.e. the filter material had not been biologically activated. The IgG solution was pumped through the 10 discs of filter material at a rate of 3 ml/minute.

Some of the IgG passes through the membrane and is observed as a peak when the effluent is monitored using a UV spectrophotometer set at 278 nm. If a peak is not observed, then all of the IgG has been absorbed and a further 10 ml of IgG is applied to the discs. The quantity of IgG in this peak can be measured by determining the area under the peak and relating it to a known amount of IgG passed through the discs without a filter being present to produce a standard peak.

After contacting with IgG, the discs are washed clean with 50 ml of PBS buffer and the spectrophotometer baseline is reestablished. IgG is removed from the membrane, more specifically from the Protein A/IgG complex by elution. For this a 0.2 M glycine/2% acetic acid solution at a pH of 3 was employed. Substantially all of the IgG was eluted, which was observed using a UV spectrophotometer. A mass balance showed that only a negligible amount of IgG was left on the membrane material. The amount of rabbit IgG removed under these "conventional" conditions was 4 mg compared to the 10 mg present in the test solution. Expressed in another way, 4 mg of rabbit IgG was separated with 1 g of membrane and 6 mg of IgG passed through, thereby substantiating the mass balance. The discs were cleaned by passage of 20 ml of PBS through them.

### Example II

This example demonstrates the use of an activated membrane in accordance with the invention in a separation procedure. It also demonstrates the optimum concentration of protector molecule to be used for forming the acceptor/protector complex. Protein A has a molecular weight of 42 000 and IgG has a molecular weight of about 160 000. A mole ratio of 1:1 would then correspond to for example 1 mg of Protein A combined with about 4 mg of IgG. A range of mole ratios were tested as shown in the following table.

The test procedure of Example I was repeated except that a mixture of Protein A and IgG with the various mole ratios was used after the activation step and the complexes bound to the membrane. 6 mg/ml Protein A was used in each test run and the IgG concentration was adjusted accordingly. For example, a mole ratio of 1:1 corresponded to 6 mg/ml Protein A and 24 mg/ml IgG. The volume of solution employed was 10 ml for the 10 discs involved.

After reacting the complex with the membrane, it was washed with a glycine/acetic acid solution at a pH of 3, washed with phosphate buffered saline (PBS) solution and then used in the affinity separation procedure. The results are indicated in the following table.

**Table 1**

| Complex Composition vs. Rabbit IgG Binding Capacity | |
|---|---|
| Complex Composition IgG/Protein A | Binding Capacity mg/g Membrane |
| 0/1 | 4.0 |
| 1/0.5 | 6.5 |
| 1/0.8 | 8.0 |
| 1/1 | 8.0 |
| 1/1.2 | 8.0 |
| 1/1.45 | 7.0 |
| 1/2.0 | 4.0 |
| 1/2.5 | 2.5 |

The results show that a broad maximum occurs in binding capacity about the molar ratio of 1:1. The capacities in the range of 1:0.5 to 1:1.5 are quite acceptable. The data also indicate that the binding capacity itself has approximately doubled when now using the acceptor/protector complex (capacity of 8 mg/g membrane) compared to the results of Example I using only Protein A (capacity of 4 mg/g membrane).

### Example III

This example was carried out to demonstrate the effects of complexing the acceptor molecule with a different type of complexing partner as the protector molecule. The acceptor was Protein A while dextran, a polysaccharide was used as the protector molecule. Dextrans of molecular weights of 40 000 corresponding to the Fc fragment of IgG and 160 000 corresponding to the molecular weight of the IgG molecule itself were tested. The higher molecular weight material was better and the following test runs made use of same. The experimental procedure was the same as in Example I. Dextran with the molecular weight of 160 000 was added to Protein A in solution at various mole ratios. 6 mg/ml Protein A was used and the amount of dextran was adjusted to give the various ratios. The volume of solution employed per 10 discs was again 10 ml. The results are given in the following table.

**Table 2**

| Dextran as the protector molecule | |
|---|---|
| Complex Composition Mole Ratio of Dextran/Protein A | Binding Capacity mg/g Membrane |
| 1/0.5 | 9.5 |
| 1/1 | 10.5 |
| 1/1.5 | 9.5 |

Unexpectedly, the binding capacity of the membrane was further increased when employing dextran as the protecting agent. The binding capacity again was high over a broad region about the ratio of 1:1.

### Example IV

This example demonstrates the effects of the manner in which the protector/acceptor complex is loaded onto the membrane. In the previous examples, the dry discs of membrane material were immersed in an aqueous solution of the complex for loading. In the present example, the dextran/Protein A complex was prepared such that the volume of the complex solution corresponded closely to the void volume of the membrane, i.e. the 10 discs to be loaded. 6 mg/ml of Protein A was employed and the amount of dextran was varied to give the respective mole ratios. The volume of solution used for the 10 discs was 2 ml. The binding capacity is given in the following table.

**Table 3**

| Void Volume Loading with Dextran/Protein A Complex | |
|---|---|
| Complex Composition Mole Ratio of Dextran/Protein A | Binding Capacity mg/g |
| 1/0.5 | 14.5 |
| 1/1 | 16 |
| 1/1.5 | 14.5 |

The results show that a further significant increase in binding capacity can be achieved with this loading technique. Compared to the immersion loading in excess solution (Example III) the binding capacity increases from 10.5 to 16 mg IgG separated per gram of membrane.

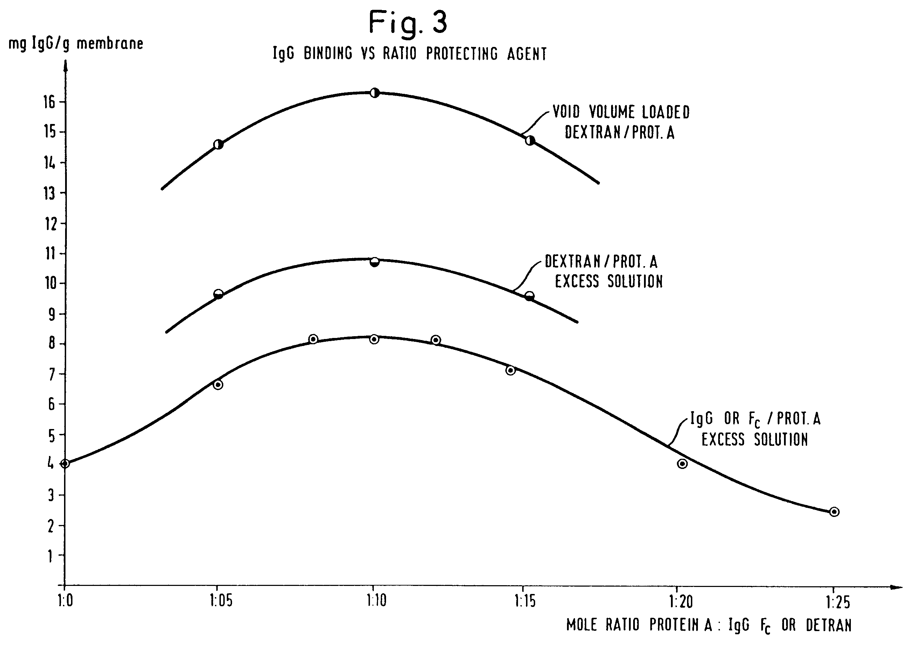

The results of Examples I, II and III are summarized in Figure 3. The data support the findings that the mole ratio of protector molecule to acceptor molecule in the range of 0.5:1 to 1:1.5 give highly satisfactory binding capacities.

The binding capacity itself is also greatly improved with the techniques of the present invention. The 16 mg/g membrane obtained with dextran/Protein A complex represents a 4-fold increase in capacity over the use of a membrane loaded with Protein A alone, i.e. not in a complex.

### Example V

This example was performed to test the effect of the pore size of the membrane on the separation capacity. A series of runs were performed again using the dextran/Protein A complex where the complex loading was performed under the immersion conditions as described in Example III. The Bio-Inert® membrane was employed with various pore ratings and the binding capacity for IgG was tested.

**Table 4**

| IgG Binding Capacity as a Function of Pore Size of the Membrane | |
|---|---|
| Pore Rating (µm) | IgG Capacity (mg) |
| 0.20 | 10 |
| 0.45 | 5 |
| 0.65 | 4 |
| 0.80 | 4 |
| 1.20 | 2 |

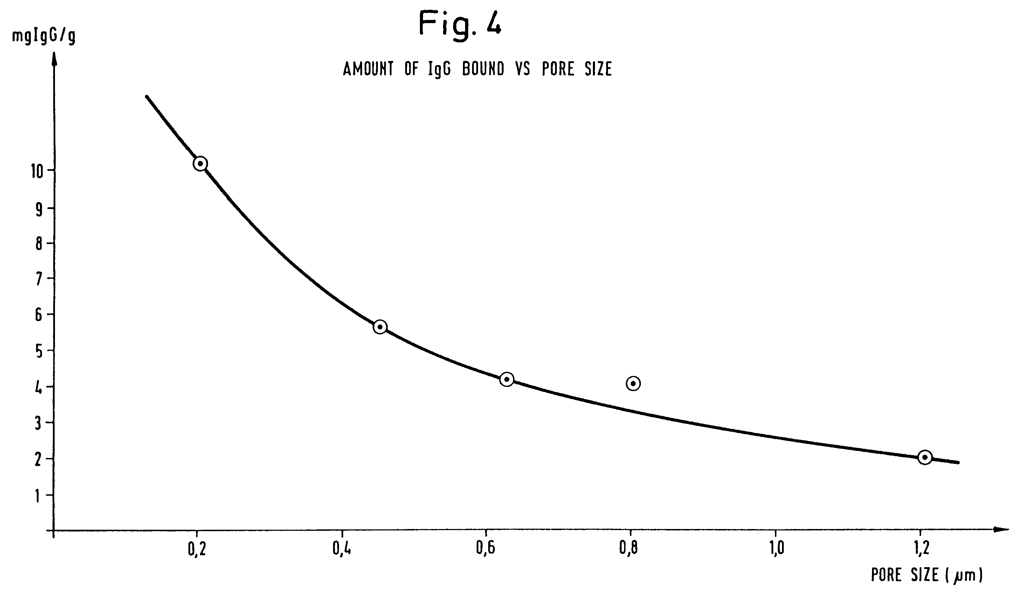

The data show that the 0.2 µm membrane as used in the previous examples gives the optimum binding capacity. Increasing the pore size and therefore decreasing the effective surface area of the membrane sharply decreases the binding capacity. The results of this example are illustrated in Figure 4. With a pore rating of greater than 0.4 µm, the binding capacity is less than half the value at 0.2 µm.

### Example VI

This example was performed to test the effect of flow rate of the ligand fluid on the binding capacity of the activated membrane. The discs of membrane material had a pore size of 0.2 µm and the material was loaded with Protein A by means of the dextran/Protein A complex. The experimental set up consisted of 10 discs of 47 mm diameter in a disc holder and a peristaltic pump capable of achieving 80 ml/minute through the system so that the IgG could be immobilized at varying flow rates.

**Table 5**

| Flow Rate Through the Activated Membrane vs. IgG Binding Capacity | |
|---|---|
| Flow Rate ml/min | IgG Bound mg/g Membrane |
| 2 | 7.5 |
| 4 | 7.5 |
| 8 | 7.5 |
| 50 | 7.0 |

The "low flow" capacity of the membrane was found to be 7.5 mg/g membrane. This capacity was nearly constant over a 25-fold increase in flow rate, indicating substantial independence of binding capacity on the flow rate.

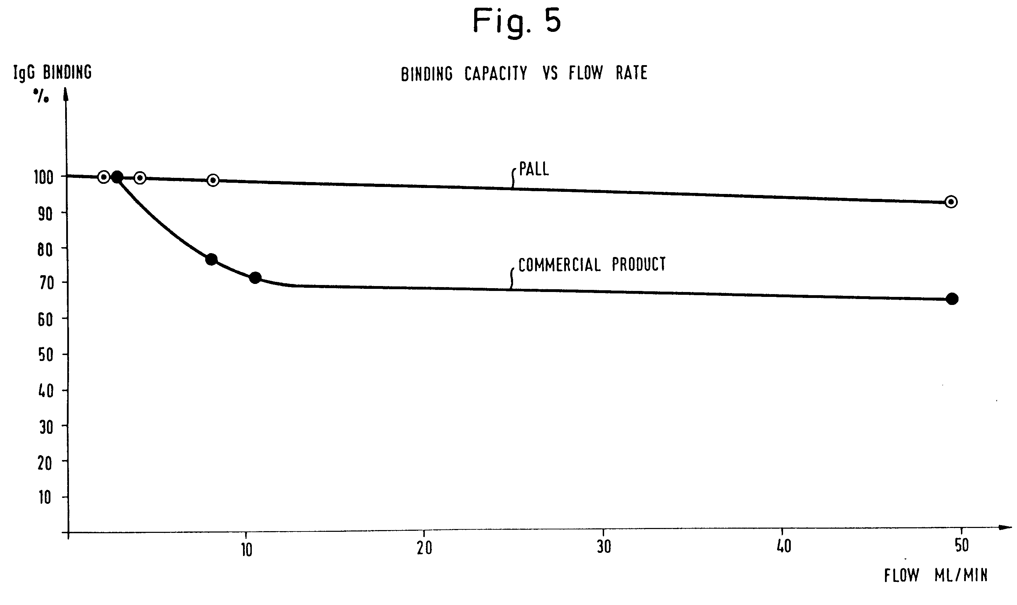

Additional comparative runs were made with a commercially available disc device. The device with membrane has a "low flow" capacity of 15 mg. To compare with the inventive runs, the results were normalized at the low flow value found at 2-3 ml/minute. The comparative run along with the runs using the inventive activated membrane are illustrated in Figure 5. At a flow rate of 10 ml/minute, the comparative device falls off by about 70% in binding capacity compared to the value at about 2 ml/minute. This indicates that the activated membrane and separation method of the present invention is less sensitive to flow rate.

## Claims

1. Method of preparing an activated or activatable membrane suitable for ligate separation comprising the steps of:
a) reacting an acceptor molecule with a protector molecule to form a complex,
b) reacting the complex of step (a) with a porous, preferably microporous, membrane having active sites capable of binding with said complex, preferably a membrane which has been formed by reacting the membrane with an activating agent to form said active sites, and
c) optionally, removing the protector molecule.

2. Method of claim 1 wherein said acceptor molecule is capable of reversibly reacting with a ligate to form a biospecific complex.

3. Method of claim 1 or 2, wherein said acceptor molecule is selected from the group of antibodies, antigenic substances, glycoproteins, Protein A, Protein G, lectins, carbohydrates, enzymes, cofactors, inhibitors, hormones, IgG class of immunoglobulins, carrier proteins, receptors, heparin, coagulation factors, histones and mimetic ligands, in particular wherein said acceptor molecule is Protein A.

4. Method of one of the preceding claims, wherein the protector molecule is selected from the group of polysaccharides, antibodies, antigenic substances, glycoproteins, Protein A, Protein G, lectins, carbohydrates, enzymes, cofactors, inhibitors, hormones, IgG class of immunoglobulins, carrier proteins, receptors, heparin, coagulation factors, histones and mimetic ligands, in particular wherein said protector molecule is a polysaccharide.

5. Method of claim one of the preceding claims, wherein the complex of step (a) is formed by reacting a mixture having a mole ratio of protector molecule to acceptor molecule in the range of 1 : 0.5 to 1: 1.5, preferably in the range 1 : 0.8 to 1 : 1.2.

6. Method of claim 4 and 5, wherein said acceptor molecule is Protein A and said protector molecule is an IgG class of immunoglobulin.

7. Method of class 4 or 5, wherein said acceptor molecule is Protein A and said protector molecule is a polysaccharide, in particular wherein said polysaccharide is a dextran.

8. Method of claim 4, 5 or 7, wherein the molecular weight of said dextran is selected to be within the range of 75 % to 125 % of the molecular weight of the ligate to be separated, in particular within the range of 90 % to 110 % of the molecular weight of the ligate to be separated.

9. Method of one of the preceding claims, wherein the step (b) of reacting the complex with the membrane further comprises contacting the membrane with a volume of solution containing the complex, said volume being in the range of 50 % to 150 % , preferably 80 to 120 % of the void volume of the membrane.

10. Method of one of the preceding claims, wherein said porous membrane is a hydrophilic, microporous, polyamide membrane, which has been surfaced modified to comprise pendant hydroxyl groups.

11. Method of claim 10, wherein the pore size rating of the polyamide membrane is within the range of 0.2 to 3.0 µm.

12. A porous membrane having attached thereto a complex of an acceptor molecule, which is capable of and designated for immobilizing a ligate, and a protector molecule which is different from the ligate and which is capable of and designated for removal from the acceptor molecule prior to the use of the membrane for ligate immobilization.

13. Porous membrane of claim 12, wherein the complex of an acceptor molecule and a protector molecule has been formed prior to attachment to said membrane.

14. Process for separating biologically separable, more particularly biologically active substances from impure solutions containing same, said process comprising
contacting said impure solution with a product, namely a separating material or device or membrane, in accordance with one of the preceding claims, thereby separating said substance from said solution by immobilizing it at said product,
and removing said remaining impure solution from said product.

15. Process in accordance with claim 14, further comprising recovering said separated substance from said product, preferably by elution.

16. Process in accordance with claim 14 or 15, comprising,
- immobilizing said substance at said product,
- removing impure solution from said product by washing said product while maintaining a substantial quantity of said substance on said product,
- eluting said product and thereby removing substance from said product, preferably obtaining a more concentrated solution of said substance in comparison with said impure solution,
- optionally laundering said product to remove unwanted and/or contaminating proteins and other alien materials from the product.

17. Process in accordance with claims 14, 15 or 16, wherein the steps are repeated, preferably in the form of 10 to 100 cycles.

18. Process in accordance with one of claims 14 to 17, characterized by employing the conditions or respectively steps of one or more of the use claims herein.

19. Process for recovering a ligate from a ligand-containing solution, particularly in accordance with one of claims 14 to 18 comprising:
a) passing said ligate-containing solution through the activated membrane prepared by the method of one of the claims 1 to 11, or through the activatable membrane of claim 12 or 13 which has been activated by removing protector molecules from the complexes,
b) binding the ligate to the acceptor molecule on the surface of said activated membrane, and
c) optionally eluting the bound ligate from said activated membrane to recover the ligand.
